Europäisches Patentamt

European Patent Office

Office européen des brevets

(11) Publication number: **0 369 078 B1**

(12) **EUROPEAN PATENT SPECIFICATION**

(45) Date of publication of patent specification: **28.07.93**  (51) Int. Cl.5: **C07C 15/08**, C07C 5/27, B01J 29/06

(21) Application number: **88310829.2**

(22) Date of filing: **16.11.88**

(54) Process for producing p-xylene and o-xylene.

(43) Date of publication of application:
**23.05.90 Bulletin 90/21**

(45) Publication of the grant of the patent:
**28.07.93 Bulletin 93/30**

(84) Designated Contracting States:
**BE DE FR GB IT NL**

(56) References cited:
**EP-A- 0 057 607**
**EP-A- 0 065 364**
**EP-A- 0 138 617**
**US-A- 3 856 872**
**US-A- 4 584 423**

(73) Proprietor: **Indian Petrochemicals Corporation Ltd.**
**P.O. Petrochemicals**
**Vadodara District Baroda 391346 Gujarat(IN)**

(72) Inventor: **Halgeri, Anand Bhimarao**
**Indian Petrochemicals Corp. Ltd.**
**P.O. Petrochem. District Vadodara**
**391346(IN)**
Inventor: **Sharma, Ramesh Chander**
**Indian Petrochemicals Corp. Ltd.**
**P.O. Petrochem. District Vadodara**
**391346(IN)**
Inventor: **Kumar, Sushil**
**Indian Petrochemicals Corp. Ltd.**
**P.O. Petrochem. District Vadodara**
**391346(IN)**
Inventor: **Bhatt, Ramesh Chandra**
**Indian Petrochemicals Corp. Ltd.**
**P.O. Petrochem. District Vadodara**
**391346(IN)**
Inventor: **Rao, Turaga Sundara Rama Prasada**
**Indian Petrochemicals Corp. Ltd.**
**P.O. Petrochem. District Vadodara**
**391346(IN)**

(74) Representative: **Collier, Jeremy Austin Grey et al**
**J.A.Kemp & Co. 14, South Square Gray's Inn**
**London WC1R 5EU (GB)**

**Description**

The present invention relates to an improved process for the catalytic isomerisation of alkyl aromatic hydrocarbons in order to produce p-xylene and o-xylene. More particularly, the invention relates to an isomerisation process for the production of p-xylene and o-xylene wherein a mixture of xylenes and ethyl benzene is reacted with hydrogen over an improved composite high-silica zeolite catalyst. This catalyst which exhibits unique catalytic behaviour for the isomerisation of xylene comprises a variable mixture of amorphous and crystalline alumina, silica and aluminosilicates.

The isomerisation of alkyl aromatics has become commercially important in view of its leading to the production of p-xylene and o-xylene. p-xylene is employed principally in the preparation of polyesters while o-xylene finds use mainly in the production of phthalic anhydride. m-xylene has fewer end-uses and accordingly is normally isomerised catalytically to the para and ortho forms which possess greater commercial application. For instance, after p-xylene and o-xylene have been separated from the isomerisation reaction, it is usual for m-xylene to be recycled to the isomerisation reactor along with any unreacted ethyl benzene and small amounts of p-xylene and o-xylene as well as any non-aromatic substances. In this way, the combined desired product and remaining alkyl aromatic hydrocarbons are recycled virtually to extinction.

Any mixture of xylenes invariably includes ethyl benzene. This creates a problem during isomerisation since ethyl benzene is not easily isomerised. Furthermore, ethyl benzene can be present in a mixed xylene stream in an amount of from 15% to 40% which is greater than the equilibrium amount.

Accordingly, it has been proposed first of all to remove ethyl benzene from the xylene mixture. Unfortunately, this is rather difficult since the boiling points of ethyl benzene and of xylenes are very close to each other. Nevertheless, it has become conventional to remove ethyl benzene present within the mixture of xylenes by means of distillation from pyrolysis gasolene. Two alternative approaches are normally taken to achieve this.

According to the first approach, the problem is handled by removing ethyl benzene by disproportionation or hydrodealkylation to yield products such as benzene and heavier $C_9$ aromatics which can be easily separated from the alkyl aromatic mixture since the latter comprises compounds having only eight carbon atoms. Examples of this sort of prior art process are those described in U.S. Patents Nos. 3856871, 3856872, 3856873 and 3856874 and in European Patent Specification 57607-A in which special zeolite molecular sieves are used to isomerise xylene while converting ethyl benzene to benzene, toluene and $C_9$ aromatics and non-aromatics. This sort of processes is known as the Mobil vapour phase isomerisation process.

As an alternative approach, the prior art discloses the reaction of ethyl benzene with hydrogen in the presence of a hydrogenation-dehydrogenation catalyst to form xylenes. Examples of such catalysts include platinum on alumina, silica alumina and a mixture of alumina and mordernite type zeolite. Typical processes of this nature are those disclosed in U.S. Patents Nos. 276332 and 4128591. The platinum based catalyst employed in such processes provides a hydrogenation-dehydrogenation function which is believed to be necessary for the conversion of ethyl benzene to xylene. Processes employing such catalyst have been found commercially successful and among such processes the one known as the 'Octafining Process' has been unique in its ability to convert ethyl benzene to xylene. Another such process having a similar effect is the one known as the 'Isomar Process'.

There is no doubt, therefore, that both the isomerisation processes of the prior art and the catalysts employed thereby have met with a fairly high degree of success not only from the point of view of isomerisation of xylenes but also from that of the conversion of ethyl benzene to xylene. Nevertheless, there is still room for improvement both from the process angle as well as from the point of view of the catalyst employed.

The prior art has disclosed a number of catalysts for the isomerisation of xylenes alone as well as for the combined isomerisation of xylenes and the conversion of ethyl benzene to xylene from a mixture of xylenes and ethyl benzene. However, the processes involved do not come anywhere near an approach to chemical equilibrium in the isomerisation of xylenes with minimum xylene loss.

This invention provides a process for the isomerisation of xylenes employing a highly selective catalyst which permits the process to be operated at substantially chemical equilibrium with minimum loss of xylenes. The process operates at very low hydrogen to hydrocarbon mole ratios at a high weight hourly space velocity, and shows a high level of tolerance for non-aromatics present among the reactants. Moreover, the catalyst employed maintains its activity and selectivity for prolonged periods.

In recent years, the art has witnessed the development of a catalyst by the National Chemical Laboratory, Pune, India or NCL as it is more popularly referred to. As a result, the catalyst in question is eponymously known as "encilite" and has represented a considerable improvement over catalysts hitherto known. The encilite catalyst, its production and use forms subject of Indian Patent Applications Nos. 159164 and 160212.

The present invention has improved on the encilite catalyst through the provision of a high silica zeolite catalyst comprising a mixture of amorphous silica and crystalline aluminosilicate preferably provided on an alumina support and incorporating minor amounts of metals of Group VIII of the Periodic Table. In an alternative form, the catalyst components can be mixed with water to form an extrudable mass which is then extruded and the extrudates then dried and calcined, for instance at 500°C for about 16 hours. By employing the improved catalyst, it is possible according to the present invention to treat an ethyl benzene containing $C_8$ aromatic fraction so as to isomerise the xylene content thereof leading to the production of p-xylene and o-xylene with the simultaneous conversion of the ethyl benzene content to benzene.

Accordingly, the present invention provides a process for the production of p-xylene and o-xylene by the catalystic isomerisation of alkyl aromatic hydrocarbons which comprises subjecting a substantially $C_8$ alkyl aromatic hydrocarbon mixture to a temperature of from 250°C to 550°C in the presence of hydrogen in a hydrogen to hydrocarbon mole ratio from 0.5 to 10 and a high silica zeolite catalyst in acid form, said catalyst comprising a mixture of amorphous silica and crystalline aluminosilicate in which the silica to alumina ratio is from 90 to 200 on an alumina support and containing small amounts of metals of Group VIII of the Periodic Table dispersed on a porous matrix whereby the xylene content of said mixture is isomerised to provide an isomeric mixture in equilibrium and the ethyl benzene present is simultaneously dealkylated, separating the reaction mixture into a lighter or top product consisting of a benzene-toluene mixture and a heavier bottoms product rich in p-xylene and o-xylene isomers, and treating said bottoms product to separate therefrom the p-xylene and o-xylene as individual products. The metal of Group VIII of the Periodic Table incorporated into said catalyst composite is preferably platinum either alone or in combination with one or more metals such as nickel or palladium.

Reaction temperature can vary but is preferably in the range of from 350°C to 500°C. The process can be effected under a pressure of 981 to 1961 kPa (10 to 20 kg/cm$^2$). More preferably, the reaction is carried out at a weight hourly space velocity (WHSV) of from 1 to 20.

In accordance with a further feature of the invention the product stream remaining after separation of p-xylene and o-xylene which is rich in $C_8$ alkylaromatic hydrocarbons is recycled to the start of the reaction. The initial alkylaromatic hydrocarbon mixture and the recycled product stream can also contain from 4% to 25% non-aromatic aliphatic hydrocarbons.

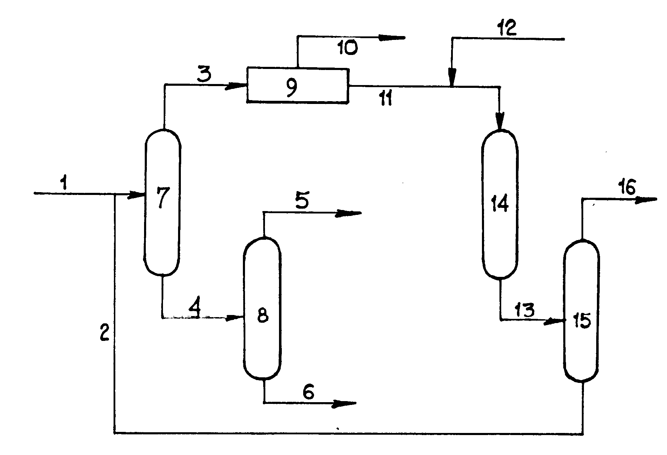

One particular embodiment of the invention is now described with reference to the accompanying drawing which illustrates a flow-diagram representative of the process of the invention.

With reference to such drawing, a mixture of $C_8$ alkyl aromatics, e.g. obtained by the distillation of a $C_5$ reformate employing a platinum catalyst dispersed over alumina, is supplied through line 1 to ortho-meta splitting column 7. After the splitting reaction is complete, a top product stream comparatively rich in p-xylene is withdrawn from column 7 via line 3 to separation unit 9. At the same time, a bottoms product comprising a mixture of o-xylene, $C_9$ aromatics and other heavy end products is led out of column 7 by line 4 to re-run tower 8.

Following treatment in tower 8, an o-xylene rich stream is withdrawn via line 5 as a top product from tower 8 while the heavier $C_9^+$ aromatics are withdrawn from the bottom of such tower through line 6. Simultaneously, p-xylene is separated from the p-xylene rich stream in unit 9 by crystallisation or selective adsorption. The separated p-xylene is led off through line 10 and the remaining $C_8$ aromatic mixture which is lean in p-xylene content and therefore ideal as isomerisation feedstock is withdrawn via line 11. The withdrawn $C_8$ mixture is mixed with hydrogen entering through line 12, heated to the appropriate temperature and introduced at reaction conditions to isomerisation reactor 14 in the presence of the isomerisation catalyst.

The essential reaction that takes place in reactor 14 is the isomerisation of the xylenes to an isomeric mixture in equilibrium. However, simultaneous secondary reactions also take place over the catalyst. These include dealkylation of ethyl benzene, disproportionation of ethyl benzene, transalkylation of xylenes and hydrocracking and indeed reaction conditions can be optimised to produce a desired product pattern. The reaction products of reactor 14 are cooled therein and then led by line 13 to isomer stabiliser 15. In stabilizer 15 the lighter products such as benzene, toluene, light saturates and gases are separated and withdrawn from the top via line 16. The residue which comprises a stream rich in $C_8$ aromatics is withdrawn as a bottoms product and recycled via line 2 as initial feed to ortho-meta splitting column 7 either directly of after blending with the fresh feed entering through line 1.

Essentially, the improved catalyst employed in the process of the present invention fulfils the optimum requirements for such a catalyst, viz. it is active to isomerise xylene and convert ethyl benzene to benzene but is inactive for generating competing reactions particularly those which destroy $C_8$ aromatics. Where an isomerisation process is involved, the activity of the catalyst is expressed in terms of approach to equilibrium of the individual isomers. The equilibrium approaches of p-xylene and o-xylene and the disappearance of ethyl benzene [EB] are calculated according to the following equations:

[i]

$$\% \text{ EB disappearance} = \frac{\text{EB in feed} - \text{EB in product}}{\text{EB in feed}} \times 100$$

[ii] % p-xylene approach to equilibrium expressed as

$$p_x\text{-ATE} = \frac{p_xP - p_xF}{p_xE - p_xF} \times 100$$

where

$p_xP$ is % p-xylene in the xylene product mixture
$p_xF$ is % p-xylene in the feed, and
$p_xE$ is % p-xylene equilibrium concentration at the reaction temperature.
[iii] % o-xylene approach to equilibrium expressed as

$$o_x\text{-ATE} = \frac{o_xP - o_xF}{o_xE - o_xF} \times 100$$

where

$o_xP$ is % o-xylene in the xylene product mixture
$o_xF$ is % o-xylene in the feed, and
$o_xE$ is % o-xylene equilibrium concentration at the reaction temperature.
The selectivity of the catalyst is expressed in terms of % xylene loss and % $C_8$ aromatics loss and these losses are represented hereafter by equations [iv] and [v], respectively:

[iv]

$$\% \text{ xylene loss} = \frac{XF - XP}{XF} \times 100$$

where

XF = total xylenes in the feed, and
XP = total xylenes in the product.

[v]

$$\% \; C_8 \; \text{aromatics loss} = $$

$$\frac{C_8AF \; - \; C_8AP}{C_8 \; A \; F} = \times \; 100$$

where

$C_8AF$ = total $C_8$ aromatics in the feed, and

$C_8AP$ = total $C_8$ aromatics in the product.

The present invention will now be described at length and in greater detail in the following examples. In such examples the selectivity of the catalyst is indicated as a percentage of xylene and $C_8$ aromatics losses. In particular, the examples underline the fact that the reaction conditions under which the invention can be conducted can vary depending on the content of the feed and on the composition desired in the final product mixture.

In Examples 1 to 4, the results of a series of test runs conducted in a pilot plant over the improved high silica zeolite catalyst in the form of 1/16 inch (1.6 mm) extrudates are set out. Conditions other than temperature were maintained constant with a pressure of 1569 kPa (16 kg/cm$^2$), a weight hourly space velocity (WHSV) of 10 and a hydrogen to hydrocarbon molar ratio of 2.0. In each instance the charge was commercial naphtha reformate.

## EXAMPLE 1

A series of three test runs were conducted in a pilot plant reactor under simulated conditions which varied the reaction temperature of each of the runs but maintained the other parameters constant as follows:

| | |
|---|---|
| Pressure | 1569 kPa (16 kg/cm$^2$), |
| WHSV | 10 |
| Hydrogen-hydrocarbon molar ratio | 2.0 |

The charge which was a commercial $C_8$ aromatic fraction of a typical naphtha reformate comprised on a weight percent basis: 9.7% p-xylene, 51.9% m-xylene, 21.7% ethyl benzene, 3.2% toluene, 7.3% saturates.

The results of liquid product analysis of each of the three runs conducted are set out below:

| | Run 1 | Run 2 | Run 3 |
|---|---|---|---|
| Temperature | 410°C | 430°C | 435°C |
| ANALYSIS wt. % | | | |
| Saturates | 6.4 | 3.69 | 3.24 |
| Benzene | 6.11 | 9.53 | 10.06 |
| Toluene | 4.2 | 4.88 | 4.6 |
| Ethyl benzene | 12.5 | 10.9 | 10.02 |
| p-xylene | 15.85 | 16.10 | 16.19 |
| m-xylene | 37.35 | 36.6 | 36.4 |
| o-xylene | 15.62 | 15.78 | 16.12 |
| $C_9A$ aromatics | 2.38 | 2.56 | 2.228 |
| Ethyl benzene Conv % | 40.0 | 48.6 | 52.4 |
| Xylene loss % | 1.44 | 1.67 | 1.88 |
| $C_8$ A Loss % | 11.5 | 12.0 | 12.5 |
| ATE p-xylene % | 100 | 100 | 100 |
| ATE o-xylene % | 92 | 95 | 96 |

The results given above indicate that the reaction temperature is a critical parameter and is particularly relevant to control ethyl benzene conversion which increases as the reaction temperature increases. It is observed that the approach of o-xylene to equilibrium will increase with the rise in temperature. A marginal increase in xylene loss and $C_8$ aromatics loss is also indicated with the rise in temperature.

EXAMPLE 2

As in Example 1, a series of three test runs were conducted in a pilot plant reactor under simulated conditions but in this instance, it was pressure that was varied in respect of each run, the other parameters being maintained constant as follows:

| Temperature | 410°C |
|---|---|
| WHSV | 10 |
| Hydrogen-hydrocarbon molar ratio | 2.0 |

The charge which was a commercial $C_8$ aromatic fraction of a typical naphtha reformate comprised on a weight percent basis: 9.36% p-xylene, 9. 7% o-xylene, 48.33% m-xylene, 21.25% ethyl benzene, 4.8% toluene, 6.56% saturates.

The results of liquid product analysis of each of the three runs conducted are set out below:

| | Run 1 | Run 2 | Run 3 |
|---|---|---|---|
| Pressure, kPa (kg/cm$^2$) | 1569 (16) | 1422 (14.5) | 1275 (13) |
| ANALYSIS wt. % | | | |
| Saturates | 6.4 | 4.00 | 4.11 |
| Benzene | 6.11 | 6.6 | 6.65 |
| Toluene | 4.26 | 4.48 | 4.46 |
| Ethyl benzene | 12.26 | 14.11 | 4.46 |
| p-xylene | 16.00 | 15.98 | 16.14 |
| m-xylene | 36.93 | 37.26 | 37.06 |
| o-xylene | 15.66 | 15.45 | 15.61 |
| $C_9$ A aromatics | 2.38 | 2.15 | 2.17 |
| Ethyl benzene Conv % | 40.3 | 32.03 | 33.75 |
| Xylene loss % | 1.44 | 1.25 | 1.29 |
| $C_8$ A Loss % | 11.5 | 9.53 | 9.7 |
| ATE p-xylene % | 100 | 100 | 100 |
| ATE o-xylene % | 92 | 88 | 90 |

The results of the three runs of this example establish that the p-xylene and o-xylene approach to equilibrium does not vary with pressure in the range study. The ethyl benzene conversion decreases with the decrease in pressure. There are marginal decreases in $C_8$ aromatics loss and xylene loss with decrease in pressure.

EXAMPLE 3

Following Example 1, a series of four test runs were conducted in a pilot plant reactor under simulated conditions but in this instance, it was the hydrogen-hydrocarbon molar ratio that was varied in respect of each run, the other parameters being maintained constant as follows:

| Temperature | 410°C |
|---|---|
| Pressure | 1569 kPa (16 kg/cm$^2$) |
| WHSV | 10 |

The charge which was a commercial $C_8$ aromatic fraction of a typical naphtha reformate comprised on a weight percent basis: 9.36% p-xylene, 9.7% o-xylene, 48.33% m-xylene, 21.25% ethyl benzene, 4.8% toluene, 6.56% saturates.

The results of liquid product analysis of each of the four runs conducted are set out below:

|  | Run 1 | Run 2 | Run 3 | Run 4 |
|---|---|---|---|---|
| $H_2$-HC molar ratio | 1 | 2 | 3 | 4 |
| ANALYSIS (wt. %) |  |  |  |  |
| Saturates | 5.44 | 6.4 | 6.33 | 6.41 |
| Benzene | 7.47 | 6.11 | 6.11 | 6.41 |
| Toluene | 4.52 | 4.26 | 4.33 | 4.23 |
| Ethyl benzene | 12.0 | 12.26 | 12.7 | 13.5 |
| p-xylene | 16.0 | 15.9 | 16.05 | 16.23 |
| m-xylene | 36.47 | 36.93 | 36.77 | 37.16 |
| o-xylene | 15.51 | 15.66 | 15.51 | 15.65 |
| $C_9A$ aromatics | 2.69 | 2.38 | 2.21 | 1.62 |
| Ethyl benzene conv. % | 43.5 | 41.5 | 38.9 | 36.4 |
| Xylene loss % | 2.3 | 1.67 | 1.44 | 0.61 |
| $C_8$ A Loss % | 12.5 | 11.5 | 11.04 | 9.65 |
| ATE p-xylene % | 100 | 100 | 100 | 100 |
| ATE o-xylene % | 92 | 92 | 92.5 | 90 |

From the results of the four runs of this example, it will be observed that the percentage of ethyl benzene conversion decreases with the increase in hydrogen-hydrocarbon molar ratio. There is no significant change in the approach to equilibrium values. However, xylene loss and $C_8$ aromatics loss are reduced with the increase in the hydrogen-hydrocarbon molar ratio.

EXAMPLE 4

As in Example 1, a series of three test runs were conducted in a pilot plant reactor under simulated conditions but in this instance, it was the weight hourly space velocity (WHSV) that was varied in respect of each run, the other parameters being maintained constant as follows:

| Temperature | 410°C |
|---|---|
| Pressure | 1569 kPa (16 kg/cm$^2$) |
| Hydrogen-hydrocarbon molar ratio | 2.0 |

The charge which was a commercial $C_8$ aromatic fraction of a typical naphtha reformate comprised on a weight percent basis: 9.36% p-xylene, 9.7% o-xylene, 48.33% m-xylene, 21.0% ethyl benzene, 4.8% toluene, 6.6% saturates.

The results of liquid product analysis of each of the three runs conducted are set out below:

7

|  | Run 1 | Run 2 | Run 3 |
|---|---|---|---|
| WHSV (hr⁻) | 10 | 9 | 8 |
| ANALYSIS wt. % |  |  |  |
| Saturates | 6.40 | 5.71 | 6.04 |
| Benzene | 6.11 | 6.82 | 6.76 |
| Toluene | 4.26 | 4.39 | 4.44 |
| Ethyl benzene | 12.26 | 12.24 | 11.90 |
| p-xylene | 16.00 | 16.04 | 16.09 |
| m-xylene | 36.90 | 36.71 | 36.74 |
| o-xylene | 15.66 | 15.73 | 15.71 |
| $C_9 A$ aromatics | 2.38 | 2.36 | 2.32 |
| Ethyl benzene Conv % | 42.3 | 42.4 | 44.0 |
| Xylene loss % | 1.67 | 1.8 | 2.0 |
| $C_8$ A Loss % | 11.5 | 11.66 | 12.0 |
| ATE p-xylene % | 100 | 100 | 100 |
| ATE o-xylene % | 92 | 95 | 96 |

The results of the runs conducted by this example establish that the percentage approach to equilibrium of p-xylene o-xylene remained more or less constant when varying the space velocity in the range of 8 to 10 whereas the ethyl benzene conversion percentage decreased marginally. Similarly, no significant change was noticed for xylene loss and $C_8$ aromatics loss in the range of WHSV that was studied.

EXAMPLES 5, 6 and 7

Three separate test runs were conducted in the isomerisation reactor of a commercial aromatics plant employing the improved high silica zeolite catalyst. In respect of each run, temperature, pressure, WHSV and the hydrogen-hydrocarbon molar ratios were maintained constant but the non-aromatics content of the feed charge was varied from run to run by introducing into a $C_8$ aromatic fraction $C_6$ to $C_8$ non-aromatic hydrocarbon material. The constant parameters were as follows:

| Temperature | 420 °C |
|---|---|
| Pressure | 1569 kPa (16 kg/cm²) |
| WHSV | 10 |
| Hydrogen-hydrocarbon molar ratio | 2.0 |

In respect of each run, the performance of the catalyst was measured. The composition of the feed charge and the results of liquid product analysis for each example/run are set out hereafter:

8

EXAMPLE 5 - Run 1

| Composition | Charge | Product Analysis wt. % |
|---|---|---|
| Saturates | 10.1 | 9.5 |
| Benzene | 0.0 | 4.6 |
| Toluene | 2.7 | 3.2 |
| Ethyl benzene | 20.2 | 12.7 |
| p-xylene | 10.5 | 16.3 |
| m-xylene | 52.2 | 35.8 |
| o-xylene | 4.2 | 16.0 |
| $C_9A$ aromatics | 0.0 | 1.9 |
| Ethyl benzene Conv % | | 39.8 |
| Xylene Loss % | | 2.5 |
| $C_8$ A Loss % | | 11.2 |
| ATE p-xylene % | | 106.5 |
| ATE o-xylene % | | 96.5 |

EXAMPLE 6 - Run 2

| Composition | Charge | Product Analysis wt. % |
|---|---|---|
| Saturates | 15.3 | 15.4 |
| Benzene | 0.0 | 3.8 |
| Toluene | 2.6 | 2.7 |
| Ethyl benzene | 19.0 | 11.9 |
| p-xylene | 9.9 | 15.3 |
| m-xylene | 50.8 | 33.3 |
| o-xylene | 2.4 | 15.0 |
| $C_9A$ aromatics | 0.0 | 2.6 |
| Ethyl benzene Conv % | | 39.4 |
| Xylene Loss % | | 2.3 |
| $C_8$ A Loss % | | 10.9 |
| ATE p-xylene % | | 108.0 |
| ATE o-xylene % | | 95.0 |

EXAMPLE 7 - Run 3

| Composition | Charge | Product Analysis wt. % |
|---|---|---|
| Saturates | 16.0 | 15.1 |
| Benzene | 0.0 | 4.2 |
| Toluene | 2.8 | 2.9 |
| Ethyl benzene | 17.8 | 11.0 |
| p-xylene | 9.2 | 15.4 |
| m-xylene | 46.9 | 33.3 |
| o-xylene | 7.3 | 15.0 |
| $C_9A$ aromatics | 0.0 | 3.1 |
| Ethyl benzene Conv % | | 40.23 |
| Xylene Loss % | | 2.82 |
| $C_8$ A Loss % | | 11.02 |
| ATE p-xylene % | | 107.69 |
| ATE o-xylene % | | 92.76 |

A comparison of the results of the runs of Examples 5, 6 and 7 shows that although the saturates in the isomer feed charge varied from 10.1% to 16%, no significant variation was observed in respect of ethyl benzene conversion or in respect of the stability of the improved zeolite catalyst. Similarly, the p-xylene and o-xylene approaches were quite satisfactory throughout the runs. An interesting observation which emerges from these three examples is that the improved catalyst can tolerate amounts of saturates in the feed as high as 10% to 16% without any adverse effect on its overall activity and stability and the selectivity of the product.

EXAMPLES 8, 9 and 10

In order to study the performance of the improved high silica zeolite catalyst employed by this invention at various severities of operation, three further test runs were conducted in the isomerisation reactor of a commercial aromatics plant employing such catalyst. The temperature for each run was varied over a range of from 400°C to 440°C, maintaining pressure, WHSV and hydrogen-hydrocarbon molar ratio constant. These constant parameters were as follows:

| | |
|---|---|
| Pressure | 1569 kPa (16 kg/cm$^2$) |
| WHSV | 10 |
| Hydrogen-hydrocarbon molar ratio | 2.0 |

In respect of each run, the performance of the catalyst was measured. The composition of the feed charge and the results of liquid product analysis for each example/run are set out hereafter:

EXAMPLE 8 - Run 1

The temperature of the operation was 405°C.

| Composition | Charge | Product Analysis wt. % |
|---|---|---|
| Saturates | 12.2 | 11.7 |
| Benzene | 0.0 | 2.8 |
| Toluene | 1.8 | 2.1 |
| Ethyl benzene | 19.5 | 13.0 |
| p-xylene | 9.5 | 16.2 |
| m-xylene | 49.7 | 35.9 |
| o-xylene | 7.3 | 16.2 |
| $C_9$ A aromatics | 0.0 | 1.9 |
| Ethyl benzene Conv % | | 33.3 |
| Xylene Loss % | | 1.3 |
| $C_8$ A Loss % | | 8.9 |
| ATE p-xylene % | | 105.0 |
| ATE o-xylene % | | 94.0 |

EXAMPLE 9 - Run 2

The temperature of the operation was 420°C.

| Composition | Charge | Product Analysis wt. % |
|---|---|---|
| Saturates | 15.5 | 14.5 |
| Benzene | 0.0 | 3.3 |
| Toluene | 2.3 | 2.9 |
| Ethyl benzene | 19.1 | 11.6 |
| p-xylene | 9.8 | 15.7 |
| m-xylene | 49.4 | 34.0 |
| o-xylene | 3.9 | 15.4 |
| $C_9$ A aromatics | 0.0 | 2.6 |
| Ethyl benzene Conv % | | 40.75 |
| Xylene Loss % | | 1.39 |
| $C_8$ A Loss % | | 8.9 |
| ATE p-xylene % | | 106.0 |
| ATE o-xylene % | | 95.0 |

EXAMPLE 10 - Run 3

The temperature of the operation was 435 °C.

| Composition | Charge | Product Analysis wt. % |
|---|---|---|
| Saturates | 9.2 | 8.82 |
| Benzene | 0.0 | 5.4 |
| Toluene | 2.3 | 3.76 |
| Ethyl benzene | 17.9 | 8.50 |
| p-xylene | 11.6 | 17.08 |
| m-xylene | 53.3 | 37.22 |
| o-xylene | 5.7 | 17.08 |
| $C_9A$ aromatics | 0.0 | 1.75 |
| Ethyl benzene Conv % | | 51.0 |
| Xylene Loss % | | 1.5 |
| $C_8$ A Loss % | | 10.6 |
| ATE p-xylene % | | 104.4 |
| ATE o-xylene % | | 97.3 |

A comparison of the results of the runs of Examples 8, 9 and 10 clearly establishes that reaction temperature controls the ethyl benzene conversion and is also a critical factor for severity of operation. In general, the severity of operation can be controlled by altering the reaction temperature. At low severity of operation around 30% ethyl benzene conversion, the formation of benzene is low but increases with increased severity at elevated temperatures. p-xylene approach is independent of the severity of operation but o-xylene approach increased with a rise in temperature. $C_8$ aromatics losses show an increasing trend with increased reaction temperature whereas xylene loss is correspondingly marginal. An interesting feature is that the rate of cracking of saturates is higher at high severity of operation and the build-up of saturates in the isomeriser loop can thus be reduced by operating the inlet reactor at elevated temperature. It is also possible to maximise xylene production by proper choice of the severity of operation.

EXAMPLE 11

In order to assess the stability of the improved high silica zeolite catalyst employed by the invention, four test runs were carried out in respect of different specimens of the catalyst which had been in operation over varying periods of time. Each of these runs was effected under identical reaction conditions, viz. a temperature of 420 °C., a pressure of 1569 kPa (16 kg/cm$^2$), a weight hourly space velocity of 10 and a hydrogen-hydrocarbon molar ratio of 2.

The results of each of the four runs conducted are set out below:

| | Run 1 | Run 2 | Run 3 | Run 4 |
|---|---|---|---|---|
| Catalyst-Hours on Stream | 750 | 1700 | 3500 | 5000 |
| ACTIVITY (wt. %) | | | | |
| Ethyl benzene conversion | 40.50 | 40.60 | 41.2 | 42.0 |
| ATE p-xylene | 105 | 104 | 106 | 105 |
| ATE o-xylene | 95 | 96 | 96 | 96 |

The results presented above demonstrate the remarkable stability of the catalyst of the invention whereby even after 5000 hours on stream under identical operating conditions, the ethyl benzene conversion was maintained at approximately 40% throughout the run in question. The p-xylene and o-xylene approaches were well above 100% and 90% respectively. The catalyst stability during commercial run was good and the rise in temperature required to maintain the same severity at the end of 10 months operation was 2 °C. to 3 °C. This indicates that the catalyst is resistant to coking and has a predicted life of more than twelve months.

12

EXAMPLE 12

In order to assess the comparative performance of the improved high silica zeolite catalyst of the invention both when employed as a fresh catalyst and after regeneration thereof, three tests were carried out in respect of the same catalyst. Each of these tests was effected under identical reaction conditions, viz. a temperature of 420°C., a pressure of 1569 kPa (16 kg/cm$^2$), a weight hourly space velocity of 10 and a hydrogen-hydrocarbon molar ratio of 2.

The results of each of the three tests conducted are set out below:

|  | Fresh Catalyst | Catalyst regenerated once | Catalyst regenerated twice |
|---|---|---|---|
| Hours on Stream | 100 | 1500 | 3000 |
| ACTIVITY (wt. %) |  |  |  |
| Ethyl benzene conversion | 41.0 | 40.0 | 39.5 |
| ATE p-xylene | 101.0 | 100.6 | 39.5 |
| ATE o-xylene | 94.0 | 93.0 | 92.0 |

The data given above establish that even after successive regenerations, the catalyst of the invention does not show any appreciable loss in activity or selectivity. This is indicative of excellent catalysts stability. The results also suggest that, based on pilot test runs, the catalyst of the invention will have a longer average cycle length than earlier known catalysts.

The foregoing description and the examples are provided for the purpose of guiding persons skilled in the art and it must be appreciated that the invention is not restricted only thereto. Other modifications and embodiments of the invention are clearly possible within the scope of what has been described herein.

**Claims**

1. A process for the production of p-xylene and o-xylene by the catalytic isomerisation of alkyl aromatic hydrocarbons which comprises subjecting a substantially C$_8$ alkyl aromatic hydrocarbon mixture to a temperature of from 250°C to 550°C in the presence of hydrogen in a hydrogen to hydrocarbon mole ratio from 0.5 to 10 and a high silica zeolite catalyst in acid form, said catalyst comprising a mixture of amorphous silica and crystalline aluminosilicate in which the silica to alumina ratio is from 90 to 200 on an alumina support and containing small amount of metals of Group VIII of the Periodic Table dispersed on a porous matrix whereby the xylene content of said mixture is isomerised to provide an isomeric mixture in equilibrium and the ethyl benzene present is simultaneously dealkylated, separating the reaction product into a lighter or top product consisting of a benzene-toluene mixture and a heavier bottoms product rich in p-xylene and o-xylene isomers, and treating said bottoms product to separate therefrom the p-xylene and o-xylene as individual products.

2. A process as claimed in claim 1 wherein the metal of Group VIII of the Periodic Table incorporated into said catalyst is platinum or a combination of platinum with one or more metals selected from nickel and palladium.

3. A process as claimed in claim 1 or 2 wherein said catalyst is employed in the form of an extrudate of the components thereof mixed with water to an extrudable mass, extruded, dried and calcined.

4. A process as claimed in any of claims 1 to 3 wherein the isomerisation reaction is effected at a temperature in the range of from 350°C to 500°C.

5. A process as claimed in any of claims 1 to 4 wherein the reaction is effected at a pressure in the range of from 10 to 20 kg/cm$^2$.

6. A process as claimed in any of claims 1 to 5 wherein the reaction is effected at a weight hourly space velocity (WHSV) of from 1 to 20.

7. A process as claimed in any of claims 1 to 6 wherein the product stream remaining after separation of p-xylene and o-xylene which is rich in C$_8$ alkylaromatic hydrocarbons is recycled to the start of the reaction.

**8.** A process as claimed in any of claims 1 to 7 wherein the starting alkylaromatic hydrocarbon mixture contains from 4% to 25% non-aromatic aliphatic hydrocarbon.

## Patentansprüche

**1.** Verfahren zur Herstellung von p-Xylol und o-Xylol durch katalytische Isomerisierung von alkylaromatischen Kohlenwasserstoffen, bei dem man eine Mischung aus im wesentlichen $C_8$-alkylaromatischen Kohlenwasserstoffen einer Temperatur von 250°C bis 550°C in Gegenwart von Wasserstoff bei einem Wasserstoff/Kohlenwasserstoff-Molverhältnis von 0.5-10 und eines Zeolithkatalysators mit hohem Siliciumdioxydgehalt in saurer Form unterwirft, wobei der Katalysator eine Mischung aus amorphem Siliciumdioxyd und kristallinem Aluminiumsilikat, in der das Siliciumdioxyd/Aluminiumoxyd-Verhältnis 90 bis 200 beträgt, auf einem Aluminiumoxydträger umfaßt, und der geringe Mengen an Metallen der Gruppe VIII des Periodischen Systems in einer porösen Matrix dispergiert enthält, wodurch der Xylolgehalt der Mischung isomerisiert wird, um eine Isomerenmischung im Gleichgewicht zu ergeben, und das vorhandene Ethylbenzol gleichzeitig dealkyliert wird, und das Reaktionsprodukt in ein leichteres oder Kopfprodukt, das aus einer Benzol-Tolnolmischung besteht, und ein schwereres Bodenprodukt, das reich an p-Xylol- und o-Xylol-Isomeren ist, trennt, und das Bodenprodukt behandelt, um das p-Xylol und o-Xylol als individuelle Produkte davon abzutrennen.

**2.** Verfahren nach Anspruch 1, dadurch gekennzeichnet, daß das Metall der Gruppe VIII des Periodischen Systems, das in dem Katalysator enthalten ist, Platin oder eine Kombination von Platin mit einem oder mehreren der Metalle ausgewählt aus Nickel und Palladium ist.

**3.** Verfahren nach Anspruch 1 oder 2, dadurch gekennzeichnet, daß der Katalysator in Form eines Extrudats seiner Komponenten, die mit Wasser zu einer extrudierbaren Masse vermischt, extrudiert, getrocknet und kalziniert werden, verwendet wird.

**4.** Verfahren nach einem der Ansprüche 1 bis 3, dadurch gekennzeichnet, daß die Isomerisierungsreaktion bei einer Temperatur im Bereich von 350°C bis 500°C durchgeführt wird.

**5.** Verfahren nach einem der Ansprüche 1 bis 4, dadurch gekennzeichnet, daß die Reaktion bei einem Druck im Bereich von 10 bis 20 kg/cm$^2$ durchgeführt wird.

**6.** Verfahren nach einem der Ansprüche 1 bis 5, dadurch gekennzeichnet, daß die Reaktion bei einer Gewichts-Stundenraumgeschwindigkeit (WHSV) von 1 bis 20 durchgeführt wird.

**7.** Verfahren nach einem der Ansprüche 1 bis 6, dadurch gekennzeichnet, daß der nach Abtrennung von p-Xylol und o-Xylol verbleibende Produktstrom, der reich ist an $C_8$-alkylaromatischen Kohlenwasserstoffen, zum Ausgangspunkt der Reaktion zurückgeführt wird.

**8.** Verfahren nach einem der Ansprüche 1 bis 7, dadurch gekennzeichnet, daß die Ausgangsmischung der alkylaromatischen Kohlenwasserstoffe 4% bis 25% an nichtaromatischem aliphatischem Kohlenwasserstoff enthält.

## Revendications

**1.** Procédé de production de p-xylène et d'o-xylène par isomérisation catalytique d'hydrocarbures alkylaromatiques qui comprend les étapes consistant à : soumettre un mélange constitué essentiellement d'hydrocarbures alkylaromatiques en $C_8$ à une température comprise entre 250 et 550°C en présence d'hydrogène dans un rapport molaire de l'hydrogène aux hydrocarbures compris entre 0,5 et 10 et en présence d'un catalyseur constitué d'une zéolite à forte teneur en silice, sous forme acide, ledit catalyseur comprenant un mélange de silice amorphe et d'aluminosilicate cristallin dans lequel le rapport de la silice à l'alumine est de 90 à 200, sur un support en alumine et contenant de petites quantités de métaux du groupe VIII de la classification périodique dispersés sur une matrice poreuse, grâce à quoi le xylène contenu dans ledit mélange est isomérisé afin de fournir un mélange isomérique en équilibre et l'éthyle benzène présent est simultanément désalkylé, à séparer le produit réactionnel en un produit de tête ou plus léger constitué d'un mélange benzène-toluène et un produit de queue plus lourd riche en isomères p-xylène et o-xylène, et à traiter ledit produit de queue afin d'en séparer le p-xylène et l'o-xylène en tant que produits individuels.

2. Procédé selon la revendication 1, dans lequel le métal du groupe VIII de la classification périodique incorporé audit catalyseur est du platine ou une combinaison de platine et d'un ou plusieurs métaux choisis entre le nickel et le palladium.

3. Procédé selon la revendication 1 ou 2, dans lequel ledit catalyseur est employé sous forme d'un produit d'extrusion constitué de ses composants mélangés avec de l'eau en une masse extrudable, extrudé, séché et calciné.

4. Procédé selon l'une quelconque des revendications 1 à 3, dans lequel la réaction d'isomérisation est effectuée à une température comprise dans une gamme allant de 350°C à 500°C.

5. Procédé selon l'une quelconque des revendications 1 à 4, dans lequel la réaction est effectuée à une pression comprise dans une gamme allant de 10 à 20 kg/cm$^2$.

6. Procédé selon l'une quelconque des revendications 1 à 5, dans lequel la réaction est effectuée à un débit massique horaire (WHSV) compris entre 1 et 20.

7. Procédé selon l'une quelconque des revendications 1 à 6, dans lequel le courant de produit restant après la séparation de p-xylène et d'o-xylène qui est riche en hydrocarbures alkylaromatiques en C$_8$ est recyclé au début de la réaction.

8. Procédé selon l'une quelconque des revendications 1 à 7, dans lequel le mélange d'hydrocarbures alkylaromatiques de départ contient de 4 à 25 % d'hydrocarbures aliphatiques non aromatiques.